# EUROPEAN PATENT APPLICATION

(11) **EP 4 375 067 A1**
(43) Date of publication of application: **29.05.2024**
(21) Application number: 22208882.5
(22) Date of filing: 22.11.2022
(51) Int. Cl.: B33Y 10/00, B33Y 30/00, C12M 1/26, B33Y 40/00, B29C 64/141, B29C 64/10

(54) **DEVICE FOR FABRICATION OF FIBERS, 3D PRINTER COMPRISING THE DEVICE AND METHOD FOR FABRICATION OF FIBERS**

(71) Applicant: biovature GmbH, 95444 Bayreuth (DE)
(72) Inventor: Ionov, Leonid, 95444 Bayreuth (DE); Synytska, Alla, 95444 Bayreuth (DE)
(74) Representative: Pfenning, Meinig & Partner mbB

(57) **Abstract**

The present disclosure relates to a device for fabrication of fibers, a 3D printer comprising the device and a method for fabrication of fibers. The device comprises a receiving unit having a receiving surface for receiving fabricated fibers; a first dispenser unit for dispensing a fluid; a motor unit and a first oscillating unit arranged alternately pivotable with respect to the receiving surface. The motor unit drives the first oscillating unit to oscillate. The first oscillating unit comprises a fiber picking unit and is configured to move the fiber picking unit alternately relative to the receiving surface between a first and a second position. In the first position, the fiber picking unit is arranged adjacent to the first dispenser unit such that fluid dispensed by the first dispenser unit contacts the fiber picking unit. The fiber picking unit is configured to form a fiber when pivoting from the first position to the second position.

## Description

The present disclosure primarily relates to a device for fabrication of fibers, to a 3D printer comprising such a device, and to a corresponding method for fabrication of fibers.

Traditional approaches for fabrication of nano- and microfibers are based on applying a stretching force to polymer solutions and melts. Examples of commonly used nano- and microfiber spinning techniques are electrospinning, centrifugal spinning and blow spinning.

These known methods have a number of disadvantages.

For example, during most of these known methods, the fibers are not deposited in an oriented and controllable manner.

Further, during electrospinning, there is a need of high voltage (in the kV range). In addition, these known methods do not provide a combination of advantages such as (i) fast (500 mm/s and more) deposition of aligned fibers on stationary substrates, (ii) deposition on the substrates with any thickness. Therefore, these methods cannot be efficiently used for biofabrication, because cells are not able to survive during long fabrication processes . Another disadvantage is that fabrication of thick (in particular more than 2 mm) samples using electrospinning and melt electrowriting is difficult because already fabricated samples generate a barrier for deposition of new fibers.

It is an object of the present invention to develop a device and/or 3D printer solving at least one of the aforementioned problems. Further, it is an object of the present invention to provide a method addressing at least one of the aforementioned problems.

The object may be achieved by a device with the features of the main claim as well as by a 3D printer and/or a method with the features of the auxiliary claims. Advantageous designs and further developments of the invention are to be deduced from the features of the dependent claims and the following description.

The proposed device for fabrication, in particular biofabrication, of fibers, in particular artificial fibers, comprises
- a receiving unit having a receiving surface for receiving fabricated fibers
- a first dispenser unit for dispensing a fluid,
- a motor unit comprising a rotating unit and a motor, wherein the motor is configured to drive the rotating unit,
   and
- a first oscillating unit arranged alternately pivotable with respect to the receiving surface and connected to the rotating unit such that the rotating unit drives the first oscillating unit to oscillate.

The first oscillating unit comprises a fiber-picking unit. The first oscillating unit is configured to move the fiber-picking unit alternately relative to the receiving surface between a first and a second position, wherein in the first position, the fiber-picking unit is arranged adjacent to the first dispenser unit such that fluid dispensed by the first dispenser unit contacts the fiber-picking unit, wherein the fiber-picking unit is configured to form a fiber when pivoting from the first position to the second position.

The first oscillating unit may be configured to move the fiber-picking unit alternately along an arc of an imaginary circle. The first position and the second position may be located on an imaginary circle having a center corresponding to the pivot axis of the first oscillating unit. The movement of the fiber-picking unit from the first to the second position may be in a first rotational direction. The movement from the second to the first position may be in a second rotational direction opposite to the first rotational movement.

The first oscillating unit may be configured to move the fiber-picking unit beyond the first position and/or beyond the second position. That is, the first oscillating unit may be configured to move the fiber-picking unit alternately between two positions that are spaced farther apart than the first and second positions as measured along the arc on which the fiber-picking unit is moved alternately.

In the present disclosure, the term fabrication can be understood as a generic term, which in particular also includes biofabrication.

The fabricated fibers may be nanofibers. Nanofibers may be defined as fibers with a fiber diameter smaller than 1000 nanometers (nm) (1 µm). The fabricated fibers may be microfibers. Microfibers may be defined as fibers with a fiber diameter smaller than 3000 nanometers (nm) (3 µm). Microfibers may be defined as fibers with a fiber diameter larger than the diameter of nanofibers. The fabricated fibers may be macrofibers. Macrofibers may be defined as fibers with a fiber diameter smaller than 1 mm. Macrofibers may be defined as fibers with a fiber diameter larger than the diameter of microfibers.

The device may be configured to produce at least 300 mm of fiber every second and/or at least 400 mm of fiber every second and/or at least 500 mm of fiber every second. This can have the advantage that the production process is significantly faster than known processes for deposition of aligned fibers on stationary surfaces with unlimited thickness. Therefore, the fabricated fibers may be usable in biofabrication, as the device allows rapid fabrication of samples with encapsulated living cells. Living cells can survive the biofabrication process.

The rotating unit may be configured to rotate at least at 0.1 rpm, preferably at least at 300 rpm, more preferably at least at 3000 rpm. The first oscillating unit may be configured to move the fiber-picking unit at least at 0.1 mm/s, preferably at least at 100 mm/s, more preferably at least at 500 mm/s and/or at least at 1000 mm/s and/or at least at 10000 mm/s and/or at most 100000 mm/s.

The device may allow for a high speed of deposition of fibers (in particular 500 mm/s and more). The device may convert one rotation of a rotating unit to two movements of the oscillating unit: left to right and right to left. That may allow deposition of two fibers when two dispensers are used - one fiber is deposited upon movement from left to right and another is deposited upon the return movement. As result, to produce the same number of fibers and compared to known methods having rotating units, a half-rate of rotating unit may be needed. Alternatively, the same rotation rate may allow fabrication of doubled number of fibers.

The fluid may be a polymer solution or a melt. For example, the fluid may comprise or may be any solution of a polymer in water or organic solvent and/or any mixture polymer solutions and/or any mixture of solutions of polymers and particles or cells of any shape and/or any melt of a polymer and/or melt of any polymer blends or and/or melt of polymer blend with particles of any shape. In particular, the fluid may be a polymer solutions or melt. For example, a polymer solution or melt may be defined by a viscosity of at least 0.1 mPa s and/or at most 10²⁰ mPa s. In a preferred embodiment, the fluid has a viscosity of at least 10³ mPa S and/or at most 10⁵ mPa s.

The conversion of rotation in oscillating movement may result in sinusoidal time dependence of angular movement rate of the oscillating unit. The movement rate may be zero at maximal deflection, when the fiber-picking unit changes direction of movement and may touch the fluid. The movement rate may be maximal when the oscillating unit is in a central position between the first and second position when the fiber is stretched. A low movement rate at maximal deflections may allow for more efficient contacting of the polymer droplet. This effect may be particularly important for spinning of polymer melts of high viscosity. The polymer droplet may be contacted at low speed to allow efficient adhesion of polymer to fiber-picking unit. The polymer droplet may be stretched at higher rate.

In one embodiment, the receiving surface and/or the receiving unit may be rotated. Structures with disordered (randomly oriented) or helically arranged or crossed at any angle fibers can be obtained by rotation of the sample and/or the receiving unit and/or receiving surface on which the fibers are deposited.

In one embodiment the device may comprise a second dispenser unit for dispensing a fluid. The second dispenser unit may be arranged adjacent to a travel path of the fiber-picking unit such that fluid dispensed by the second dispenser unit can contact the fiber-picking unit when the fiber-picking unit is in a third position. The travel path of the fiber-picking unit may be defined by the path along which the fiber-picking unit moves back and forth during the oscillating motion. The third position can be equal to the second position or can be a different position. In one embodiment, the third position may be at an inflection point where the oscillating unit changes direction of motion. In this way, the longest possible fiber may be generated.

When two dispensers located at opposite sides are used, the device may allow deposition of two kinds of fibers.

The fiber-picking unit may be configured to deposit the formed fibers on the receiving surface. The fiber-picking unit may deposit the fibers in an orderly manner. The fiber-picking unit may deposit the fibers essentially in parallel to each other.

In one embodiment, the fiber-picking unit is a rod.

The fiber-picking unit, in particular the rod, may comprise at least one surface having a multitude of protruding pins (111) and/or needles and/or cones. The pins and/or needles and/or cones may facilitate attachment of fluid. The diameter of each pin and/or needle and/or cone can range from 0.0001 mm to 1 mm. The diameter of the pin and/or needle and/or cone may be at least 0.001 mm, preferably at least 0.2 mm. The diameter of the pin and/or needle and/or cone may be at most 1 mm, preferably at most 0.8 mm, more preferably at most 0.7 mm. The length of the pins ranges from 0.0001 mm to 100 mm.

The fiber-picking unit may have various sections. The fiber-picking unit may comprise one or more sections. The sections may have the same or different shapes. For example, a section may have a circular and/or triangular and/or tetragonal and/or square and/or a polygonal of n sides, wherein n is an integer from 3 to 12, cross section.

The diameter and/or thickness of the fiber-picking unit can range from 0.001 mm to 10 mm. The diameter and/or thickness of the fiber-picking unit may be at least 0.01 mm, preferably less than 2 mm, more preferably less than 1 mm. The diameter and/or thickness of the fiber-picking unit may be at most 10 mm, preferably at most 9 mm, more preferably at most 7 mm. A smaller diameter may have the advantage of less vibrations. In particular, when one fiber-picking unit has two ends each connected to an oscillating unit, respectively, the diameter may be less than a diameter of a fiber-picking unit being connected only at one end to an oscillating unit. In particular, the diameter and/or thickness of a fiber-picking unit being connected only at one end to an oscillating unit may be at least 0.1 mm or at least 0. 5 mm or at least 1 mm. The diameter and/or thickness of a fiber-picking unit being connected only at one end to an oscillating unit may be at most 2 cm or at most 1.5 cm or at most 1 cm. The preferable diameter is 1 - 2 mm. In particular, the diameter and/or thickness of a fiber-picking unit being connected at least two ends to at least one oscillating unit each may be at least 0.01 mm or at least 0.05 mm or at least 0.1 mm. The diameter and/or thickness of a fiber-picking unit being connected at least two ends to at least one oscillating unit each may be at most 1 mm or at most 0.75 mm or at most 0.5 mm.

The length of fiber-picking unit can be from a few millimeters to a few meters. The length of fiber-picking unit may be at least 1 mm, preferably at least 20 mm, more preferably at least 30 mm. The length of fiber-picking unit may be at most 1 m, preferably at most 0.5 m, more preferably at most 0.2 m. Preferably, the length is a 3 - 50 cm.

The first dispenser unit and/or the second dispenser unit comprise(s) at least one nozzle for dispensing the fluid. The first dispenser unit and/or the second dispenser unit may comprise a multitude of nozzles.

The multitude of nozzles may be arranged adjacent to a travel path of the fiber-picking unit. The travel path may be an imaginary surface. The multitude of nozzles may be arranged adjacent to or on the imaginary surface. The imaginary surface may be defined by the oscillating motion of the fiber-picking unit. The imaginary surface may be the surface that the fiber-picking unit traverses during the oscillating motion. For example, if the fiber-picking unit is a rod, the imaginary surface has a width that corresponds to the length of the rod. The surface may be curved. For example, the surface may be a cutout of a circumferential surface of a cylinder whose longitudinal axis may be the axis of rotation about which the oscillating unit oscillates at least partially.

A multitude of nozzles may be arranged adjacent to the imaginary surface, such that fluid dispensed by the nozzles can contact the fiber-picking unit as the fiber-picking unit passes by. At least some of the multitude of nozzles may be arranged in rows. In one embodiment, at least some of the multitude of nozzles are arranged essentially along an imaginary line extending essentially in parallel to a longitudinal axis of the rod. The arrangement of the nozzles can be adjusted to control the length and/or diameter of the fabricated fibers. Several fibers of the same length and/or same diameter can be produced and/or fibers of different lengths and/or diameters can be produced. The arrangement of the nozzles may allow determine a location of a fabricated fiber on the receiving surface. For example, the shorter a distance between the nozzle and the turning point of the pivoting movement, the thicker and/or shorter the fiber can be generated. Additionally or alternatively, the droplet quantity of fluid provided by the nozzle may be adjusted.

In one embodiment, electric voltage may be applied between the fiber-picking unit and the first and/or second dispenser unit to stimulate transfer of fluid from the first and/or second dispenser unit to the fiber-picking unit.

The number of nozzles which dispense fluid, for example polymer solution and/or melt, can be one or more. Each nozzle can be connected to one or several dispensers. Different nozzles can be connected to the same or different dispensers. The droplet of fluid can be located above or below the fiber-picking unit. The fiber-picking unit may touch a droplet from its bottom or from the top or from the side. A microfluidic setup can be used for mixing and delivering liquid to the dispenser. The dispensers and/or nozzles may provide different fluids, for example, to provide a multi component fiber. At least two nozzles may provide two different fluids to generate a two component fiber. Additionally or alternatively, at least three nozzles may provide two different fluids to generate a two component fiber. Additionally or alternatively, at least a multitude of nozzles may provide a multitude of different fluids to generate a multi component fiber.

The rotating unit may have a circular and/or triangular and/or tetragonal and/or square and/or a polygonal of n sides shape. n may be an integer from 3 to 12. The diameter of the rotating unit can range from few millimeters to few meters. Preferably, the diameter has a size of few centimeters. The diameter may be at least 2 mm or at least 10 mm. The diameter may be at most 1 m or at most 0.5 m. For example, the diameter is in the range of 1 cm to 5 cm. The thickness of the rotating unit can vary in the range from a few millimeters to a few centimeters. Preferably, the width and/or the thickness is a few millimeters. The thickness may be at least 0.1 mm or at least 1 mm. The thickness may be at most 10 cm or at most 1 m. For example, the thickness is in the range of 2 mm to 5 mm.

The first oscillating unit may have an elongated shape. The first oscillating unit may have a rod-like shape. The oscillating unit may be a single body object or may consist of several connectable elements. One of the elements may be an elongated rod. The length of oscillating unit can vary in the range of a few millimeters to a few meters. In a preferred embodiment, the length of the first oscillating unit may be at least 10 mm, preferably at least 50 mm, more preferably at least 100 mm. In a preferred embodiment, the length of the first oscillating unit may be at most 5 m, preferably at most 0.1 m, more preferably at most 0.5 m. For example, the length is in the range of 5 cm to 15 cm. Thus, a preferred length may be few centimeters or meters. The width and thickness of oscillating unit can vary in the range of a less than a millimeter to a few centimeters. Thus, a preferred width and thickness may be from 0.1 mm to a few millimeters. The width may be at least 0.1 mm or at least 1 mm. The width may be at most 10 cm or at most 50 cm. The thickness may be at least 0.1 mm or at least 1 mm The thickness may be at most 20 cm or at most 10 cm.

The device may be configured to transmit a rotational movement of the motor unit to the first oscillating unit in such a way that the latter pivots back and forth alternately. A transmission of a rotational movement of the motor into an alternating pivoting movement of the first oscillating unit can be achieved by means of a guiding slot and a pin slidably arranged therein.

An alternating pivoting movement of the first oscillating unit can be defined in particular as a movement of the first oscillating unit back and forth about an axis of rotation along a circular arc that has the axis of rotation as its center. In particular, the circular arc can be described by a covered angle ϕ. ϕ may be at least 10 ° or at least 20° or at least 30 ° or at least 40 °. ϕ may be at most 180 ° or at most 150° or at most 120 ° or at most 100 °. In a preferred embodiment the angle ϕ may be in the range of 120° to 150°. For example, a larger angle ϕ may result in a shorter oscillating unit. As result, a higher oscillation speed may be achievable. Additionally or alternatively, a larger angle ϕ may reduce noise and/or vibrations.

The guiding slot may be arranged in the first oscillating unit. The length of the guiding slot can be the same as the length of oscillating unit or may be smaller. The width of the guiding slot can be from a few millimeters to centimeters. Preferably, the width of the guiding slot is a few millimeters. The width may be at least 0.1 mm or at least 1mm. The width may be at most 10 mm or at most 50 mm. In particular, in a preferred embodiments the width may be in the range of 1 mm to 8 mm. The guiding slot may be straight and/or bent and/or hemispherical.

In one embodiment, the first oscillating unit may comprise a guiding slot. The guiding slot may be an essentially linear guiding slot. A pin may be slidably arranged in the guiding slot. In one embodiment, the pin may be fixed to a stationary part of the device or the pin may be fixed to a stationary part of the motor unit. In another embodiment, the pin may be fixed to the rotating unit of the motor unit.

In one embodiment, the first oscillating unit may be rotatably fixed to the rotating unit or rotatably fixed to a part of the device or the pin may be rotatably fixed to a part of the motor unit. The first oscillating unit may comprise a pin slideably arranged in a semicircular guiding slot.

The semicircular guiding slot may be disposed in a stationary part of the device, for example, a stationary part of the motor unit or a part fixed to the receiving unit. By guiding the pin in the guiding slot, a rotary motion of the rotating unit can be converted into an alternating, pivoting motion of the oscillating unit.

In one embodiment, the device may comprise a first connecting strut having a first end rotatably fixed to the first oscillating unit and a second end rotatably fixed to the rotating unit.

The connecting strut may have an elongated shape. For example, the connecting strut is a rod. The length of the connecting strut can vary in the range of a few millimeters to a few meters. For example, the connecting strut has a length of few centimeters or decimeters. The length may be at least 5 mm or at least 20 mm. The length may be at most 50 cm or at most 20 cm. In a preferred embodiment, the length may be in the range of 5 cm to 10 cm. The width and thickness of connecting unit can vary in the range of a few millimeters to few centimeters. The width may be at least 0.5 mm or at least 5 mm The width may be at most 10 mm or at most 20 mm The most preferred width and thickness are a few millimeters. In a preferred embodiment, the with and/or the thickness may be in the range of 3 mm to 8 mm.

A deflection angle of the oscillation unit can be adjustable. The rotating unit may comprise at least one adjustable joint connecting the rotating unit to the first oscillating unit and/or to the first connecting strut for varying a minimal distance between the adjustable joint and a rotating axis of the rotating unit. The first oscillating unit may comprise at least one adjustable joint connecting the rotating unit to the first oscillating unit for varying a minimal distance between the adjustable joint and the fiber-picking unit.

The rotating unit may comprise a second adjustable joint for balance. The first oscillating unit may comprise a second adjustable joint for balance.

An oscillation radius can be controlled by gluing the fiber-picking unit at different distances from center of oscillation/rotation of the oscillating unit.

The device may comprise a second oscillating unit. The second oscillating unit may be configured analogous to the first oscillating unit. The fiber-picking unit may be connected to the first and the second oscillating unit. Gravity or movement may deform the fiber picking-unit. A connection to the first and/or the second oscillating unit may provide support for the fiber-picking unit. This may allow the fabrication of larger samples and/or may decrease weight/thickness of fiber-picking unit.

The fiber-picking unit can extend between the first and second oscillating units. The fiber-picking unit may be arranged substantially perpendicular to a longitudinal axis of the first oscillating unit. The fiber-picking unit may be arranged substantially perpendicular to a longitudinal axis of the second oscillating unit.

The fibers may be deposited by the fiber-picking unit, which may be fixed by its both ends so that the fibers may be deposited only from the top of the sample. Thus, the device may allow fabrication of samples with lengths ranging from several µm to 1 m. A preferred length may be in the range of 5 cm to 20 cm.

In one embodiment, the fiber-picking unit may be a string. The string may be spanned between the first and second oscillating units. The string may be a thread or a wire. The first and second oscillating units may each comprise at least one tube for receiving and guiding the string.

Fiber picking unit can also be a metal and/or polymer fiber and/or yarn and/or filament.

The device may comprise a second connecting strut, wherein the rotating unit is a shaft and the second connecting strut is rotatably connected to the shaft. The fiber-picking unit may have a first end connected to the first oscillating unit and a second end connected to the second oscillating unit.

In one embodiment, the device may comprise a second oscillating unit and a second connecting strut, wherein the second oscillating unit and the second connecting strut are rotatably connected. The rotating unit may be a crankshaft and the first and second connecting struts may be each rotatably connected to the crankshaft to move in antiphase or in phase.

When two oscillating units moving in antiphase are connected to a crankshaft which is rotated by the motor unit, vibrations may be reduced.

In one embodiment, the device may comprise a third and/or a fourth oscillating unit each connected to the rotating element via a connecting strut. The moments of inertia of the first oscillating unit and of the third oscillating unit and/or of the second oscillating unit and of the fourth oscillating unit may be essentially the same to reduce vibrations.

The present disclosure further proposes a 3D printer comprising a device as described above. The 3D printer may be a 3D bioprinter. A 3D bioprinter may be a computer-controlled printer to produce bioarrays or tissue from previously grown individual cells. The bioprinter may comprise a sample stage and at least one extruder for applying material, for example a polymer gel with living cells encased, onto the sample stage. The sample stage may have a receiving surface that corresponds to the receiving surface of the device as described above. The material may be applied layer by layer or by droplets. The at least one extruder may be movable with regard to the receiving surface. Additionally or alternatively, the receiving surface may be movable with regard to the extruder. The oscillating unit may move with regard to the sample stage. The fiber-picking unit may deposit fibers on the top of a sample located on the receiving surface. The receiving surface may be rotatable to allow deposition of fibers in different directions. A force sensor may be integrated in the receiving surface to measure mass of deposited fibers and control the fiber spinning process.

The obtained aligned nano-micro-macro-sized fibers can be used as scaffold for cell culturing. This may be used for biofabrication of skeletal and cardiac muscle tissues as well as for other tissues with fibrous structure such as skin or uniaxial aligned cells. To this end, cells can be adsorbed or printed on the surface of layer formed by fibers. Several alternating layers of fibers and cells can be deposited. The fabricated structure can be matured in a bioreactor.

The present disclosure further relates to a method for fabrication of fibers, in particular artificial fibers. The proposed method comprises the following steps:
Providing a device according to the above description,
Pivoting the fiber-picking unit to the first position in a first direction adjacent to the first dispenser unit so that the fluid dispensed from the first dispenser unit contacts the fiber pickup unit,
Pivoting the fiber-picking unit to the second position in a second direction opposite to the first direction, thereby forming a fiber and depositing the formed fiber on the receiving surface.

The fiber may be formed by stretching the fluid into a fiber.

The features described above in relation to the device can be applied to the method by analogy.

Further objects, advantages, and features will become more apparent upon reading of the following non-restrictive description of illustrative embodiments thereof, given by way of example only with reference to the accompanying drawings.

In the appended drawings:
- Fig. 1a: is a schematic drawing of a device for fabrication of fibers based on oscillating movement in a perspective view;
- Fig. 1b: shows a schematic drawing of a device according to Fig. 1a, wherein only one oscillating unit is provided;
- Fig. 2: shows a schematic drawing of an exemplary embodiment of a fiber-picking unit to be used in a device according to one of the previous or following Figures;
- Fig. 3a: shows a schematic drawing of a first exemplary embodiment of a fiber-picking unit, an oscillating unit and a motor unit for use in a device according to Figures 1a or 1b;
- Fig. 3b: shows a schematic drawing of a second exemplary embodiment of a fiber-picking unit, an oscillating unit and a motor unit for use in a device according to Figures 1a or 1b;
- Fig. 3c: shows a schematic drawing of a third exemplary embodiment of a fiber-picking unit, an oscillating unit and a motor unit for use in a device according to Figures 1a or 1b;
- Fig. 3d: shows a schematic drawing of a fourth exemplary embodiment of a fiber-picking unit, an oscillating unit and a motor unit for use in a device according to Figures 1a or 1b;
- Fig. 4a: shows a schematic drawing of a first variant for use in a device according to Figures 1a or 1b comprising a connecting strut between the oscillating unit and rotating unit;
- Fig. 4b: shows a schematic drawing of a second variant for use in a device according to Figures 1a or 1b comprising a connecting strut between the oscillating unit and rotating unit;
- Fig. 4c: shows a schematic drawing of third variant for use in a device according to Figures 1a or 1b comprising a connecting strut between the oscillating unit and rotating unit;
- Fig. 5a: shows an exemplary rotating unit;
- Fig. 5b: shows an exemplary oscillating unit;
- Fig. 6a: shows an exemplary embodiment having two oscillating units;
- Fig. 6b: shows an exemplary assembly of two oscillating units,
- Fig. 7a: shows schematically a device essentially according to the previous figures, comprising a crankshaft;
- Fig. 7b: shows schematically a device essentially according to the previous figures, comprising a crankshaft;
- Fig. 8a: shows schematically a device essentially according to the previous figures, comprising a crankshaft and two oscillating units moving in phase;
- Fig. 8b: shows schematically a device essentially according to the previous figures, comprising a crankshaft and four oscillating units;
- Fig. 8c: shows schematically a device essentially according to the previous figures, comprising a crankshaft and two oscillating units;
- Fig. 9: shows a 3D printer comprising a device according one of the previous figures;
- Fig. 10: shows an exemplary method for producing a product with integrated fibers
- Fig. 11: shows examples of produced fibers.

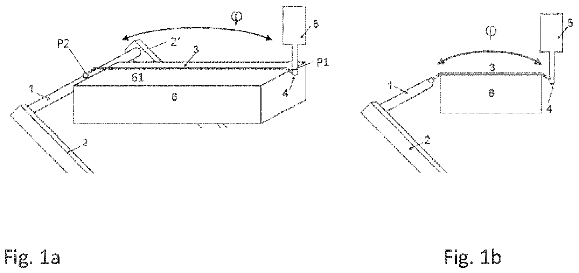

In Fig. 1a, a device for fabrication of fibers, in particular of artificial fibers is shown. A fiber-picking unit 1 is connected to a first oscillating unit 2 and a second oscillating unit 2'. The first and second oscillating units 2, 2' are elongated and each have two ends. The first and second oscillating units 2, 2' are fixedly connected by the fiber-picking unit 1 extending between a distal end of the first oscillating unit 2 and a distal end of the second oscillating unit 2'. A proximal end of the first oscillating unit 2 is not shown. A proximal end of the second oscillating unit 2' is not shown. The proximal end is located closer to the axis of rotation, wherein the distal end is located away from the axis of rotation. The first oscillating unit 2, the second oscillating unit 2' and the fiber-picking unit 1 pivot forth and back between a first position P1 and a second position P2 along a circular arc defined by angle ϕ with regard to a receiving unit 6. In the shown embodiment, the axis of rotation is located below the receiving unit 6. The receiving unit 6 may be stationary or at least partly rotatable. A dispenser unit 5 for dispensing a fluid 4 is arranged at a distance to the receiving unit. In the present embodiment, the dispenser 5 is located above the receiving unit 6. When the oscillating unit is in the first position P1, the fiber-picking unit 1 is arranged adjacent to the first dispenser unit 5 such that fluid 4 dispensed by the first dispenser unit 5 contacts the fiber-picking unit 1. When moving from the first position P1 to the second position P2, the fiber-picking unit 1 forms a fiber 3 and deposits the formed fiber 3 on a receiving surface 61 of the receiving unit 6. The oscillating units 2 and 2' are driven to move back and forth between first and second positions P1 and P2 by a motor unit (not shown). Thus, the movement of the fiber-picking unit 1 from the first position P1 to the second position P2 is in a first rotational direction and the movement from the second position P2 to the first position P1 is in a second rotational direction opposite to the first rotational movement. The fiber-picking unit 1 is a rod having a circular diameter. In other embodiments, the fiber-picking unit may have a different shape. The fiber-picking unit 1 moves repeatedly from the first position P1 to the second position P2 and back to the first position P1 and so forth. The dispenser unit 5 may continuously dispense fluid 4 and/or repeatedly dispense droplets of fluid 4 such that the fiber-picking unit 1 generates a fiber 3 each time it moves from the first position P1 to the second position P2. The fibers 3 are deposited solely on the top of the receiving surface 61 or on or next to the previously produced fiber 3. Force sensors and one or more cameras may be integrated in the receiving unit where the fibers are deposited to monitor the process of fiber deposition.

The fiber-picking unit 1 and/or the dispenser unit 5 may be arranged such that, in the first position, the fluid provided by the dispenser unit 5 remains attached to an end of the fiber-picking unit 1 (as shown, for example, in Fig. 1b). Alternatively or additionally, the dispenser unit 5 can be arranged in such a way that the fluid 4 touching the fiber-picking unit 1 contact a central area on the fiber-picking unit 1. For example, if the fiber-picking unit 1 is designed as a rod, a contact area may be located, in particular centrally, between a first end and a second end of the rod (as shown in Fig. 1a).

The proper movement of fiber-picking unit 1 is achieved by conversion of a rotation movement in oscillating /swinging movement. The rotation movement may have a rotation speed from 0.1 rpm to 2000 rpm, in some examples even up to 10000 rpm. In the present case, the rotation speed may be, for example, 300 rpm. The oscillating /swinging movement may have an average speed from 1mm/s to 100000 mm/s. In the present case, the oscillating /swinging movement may have an average speed of, for example, 1000 mm/s. In another example, the oscillating /swinging movement may have an average speed being in the range of, for example, 1 - 3m/s. This allows very fast change of direction of movement of the oscillating units 2, 2' and an accelerating/decelerating movement because of the change of direction. The accelerating/decelerating movement is advantageous for picking of polymer solution/droplet.

Figure 1b shows a device which is essentially the same as in figure 1, wherein only one oscillating unit is provided.

In the figures, recurring features are given the same reference signs.

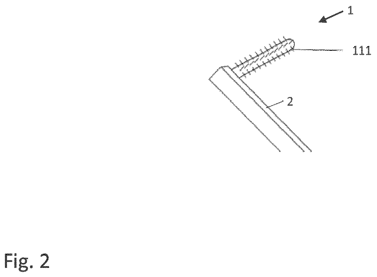

Fig. 2 shows a schematic drawing of an embodiment of a fiber-picking unit 1. The fiber-picking unit 1 has a rod shape. The fiber-picking unit 1of Fig. 2 comprises a multitude of needles 111 protruding from a surface of the rod. In Fig. 2, the needles 111 may be arranged in four rows extending along the longitudinal axis of the rod. However, in different embodiments, the needles 111 may be staggered or completely disordered. The needles 111 can improve adhesion between the fluid 4 dispensed from the dispenser unit 5 to the fiber-picking unit 1. In addition or as an alternative to needles 111, cones and/or nubs and/or pins can be provided which can improve adhesion. The needles 111 and/or cones and/or nubs and/or pins can be made of the same material as the fiber-picking unit or of a different material.

The dispenser unit 5 may comprise more than one nozzle to dispense fluid. Each nozzle may dispense the same or another fluid. Some nozzles can dispense the same liquid and one or more nozzles can dispense a different liquid. This can have the advantage of generating a structure with fibers comprising different materials and characteristics. The local arrangement of the corresponding nozzle can determine a local arrangement of the corresponding fiber in the structure to be created. Additionally or alternatively, several dispenser units 5 can be provided. Different nozzles can be connected to the same or different dispensers 5. Microfluidic setup can be used for mixing and delivering fluid 4 to the dispenser unit 5. An electric voltage may be applied between the fiber- picking unit 1 and dispenser 5 to stimulate transfer of fluid to the fiber-picking unit 1.

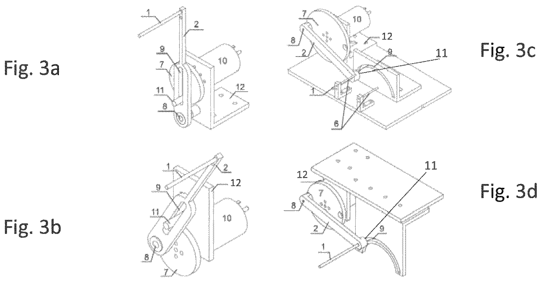

Fig. 3a shows a schematic drawing of a first exemplary embodiment of a fiber-picking unit 1, an oscillating unit 2 and a motor unit for use in a device according to Figure 1a or 1b. The oscillating unit 2 has an elongated shape extending along a longitudinal axis. A guiding slot 9 is formed in a lower area of the oscillating unit 2. The guiding slot 9 is essentially linear and extends in parallel to the longitudinal axis of the oscillating unit 2. The motor unit comprises a motor 10 and a rotating unit 7. The motor is configured to drive the rotating unit 7 to rotate around an axis of rotation. The rotating unit 7 is rotatably mounted to the motor and/or a motor holder 12. The motor 10 is fixed to the stationary motor holder 12. The oscillating unit 2 is movably fixed to the motor holder 12 via a flexible joint 8. The flexible joint 8 is arranged at a lower end of the oscillating unit 2. The oscillating unit 2 is pivotable with regard to the motor holder 12 at least partially around an axis of rotation that is parallel to the axis of rotation of the rotating unit. The fiber-picking unit 1 is fixed to an upper end of the oscillating unit 2. The rotating unit 7 comprises a pin 11 protruding into the guiding slot 9. The pin 11 is slidably arranged in the guiding slot 9. The motor 10 drives the rotating unit 7 to rotate. Due to rotation of the rotating unit 7, the pin slides in the guiding slot such that the fiber-picking unit 1 fixed to the oscillating unit 2 is pivoted alternately from the first position P1 to the second position P2 and from the second position P2 to the first position P1.

Fig. 3b shows a schematic drawing of a second exemplary embodiment of a fiber-picking unit 1, an oscillating unit 2 and a motor unit for use in a device according to Figures 1a or 1b. The oscillating unit 2 has an elongated shape extending along a longitudinal axis. A guiding slot 9 is formed in a lower area of the oscillating unit 2. The guiding slot 9 is essentially linear and extends in parallel to the longitudinal axis of the oscillating unit 2. The motor unit comprises a motor 10 and a rotating unit 7 having an axis of rotation. The motor 10 is configured to drive the rotating unit 7 to rotate around the axis of rotation. The rotating unit 7 is rotably mounted to the motor 10 and/or a motor holder 12. The motor 10 is fixed to the stationary motor holder 12. The oscillating unit 2 is movably fixed to the rotating unit 7 via a flexible joint 8. The flexible joint 8 is located at a distance to the rotating axis of the rotating unit 7. The oscillating unit 2 is pivotable with regard to the rotating unit at least partially around an axis of rotation that is parallel to the axis of rotation of the rotating unit 7. The fiber-picking unit 1 is fixed to an upper end of the oscillating unit 2 and extends essentially perpendicular with regard to the longitudinal axis of the oscillating unit 2. The motor holder 12 comprises a stationary pin 11 protruding into the guiding slot 9. The motor 10 drives the rotating unit 7 to rotate. Due to rotation of the rotating unit 7, the oscillating unit 2 is pivoted. This movement is guided by the pin 11 arranged in the guiding slot 9. The guiding slot 9 slides along the pin 11 such that the fiber-picking unit 1 fixed to the oscillating unit 2 is pivoted alternately from the first position P1 to the second position P2 and from the second position P2 to the first position P1.

Fig. 3c shows a schematic drawing of a third exemplary embodiment of a fiber-picking unit 1, an oscillating unit 2 and a motor unit for use in a device according to Figures 1a or 1b. The motor unit comprises a stationary motor 10 fixedly mounted on a stationary motor holder 12. The motor 10 drives a rotating unit 7 rotating around an axis of rotation. The oscillating unit 2 has an elongated shape. The oscillating unit 2 is pivotably fixed to the rotating unit 7 via a flexible joint 8 at a first end of the oscillating unit 2. The flexible joint 8 is located distanced to the axis of rotation of the rotating unit 7. At a second end of the oscillating unit 2, a pin 11 protrudes essentially perpendicular to a longitudinal axis of the oscillating unit 2. Further, the fiber-picking unit 1 extends from the second end of the oscillating unit 2. The fiber-picking unit extends essentially in line with the pin 11. A semicircular guiding slot 9 is arranged in a plate extending from the motor holder 12. The pin 11 is slidably arranged in the guiding slot 9. Due to rotation of the rotating unit 7, the oscillating unit 2 is pivoted. This movement is guided by the pin 11 arranged in the guiding slot 9. The pin 11 slides along the semi-circular guiding slot 9 such that the fiber-picking unit 1 fixed to the oscillating unit 2 is pivoted alternately from the first position P1 to the second position P2 and from the second position P2 to the first position P1.

Fig. 3d shows a schematic drawing of a fourth exemplary embodiment of a fiber-picking unit, an oscillating unit and a motor unit for use in a device according to Figures 1a or 1b. The motor unit comprises a stationary motor 10 fixedly mounted on a stationary motor holder 12. The motor holder 12 is a plate. The motor is fixed and arranged below the motor holder 12. The motor 10 drives a rotating unit 7 rotating around an axis of rotation. The oscillating unit 2 has an elongated shape. The oscillating unit 2 is pivotably fixed to the rotating unit 7 via a flexible joint 8 at a first end of the oscillating unit 2. The flexible joint 8 is located distanced to the axis of rotation of the rotating unit 7. At a second end of the oscillating unit 2, a pin 11 protrudes essentially perpendicular to a longitudinal axis of the oscillating unit 2. Further, the fiber-picking unit 1 extends from the second end of the oscillating unit 2. The fiber-picking unit extends essentially in line with the pin 11. A semi-circular guide slot 9 is arranged in a plate extending downward substantially perpendicular to the plane of the plate of the motor holder 12. The pin 11 is slidably arranged in the guiding slot 9. Due to rotation of the rotating unit 7, the oscillating unit 2 is pivoted. This movement is guided by the pin 11 arranged in the guiding slot 9. The pin 11 slides along the semi-circular guiding slot 9 such that the fiber-picking unit 1 fixed to the oscillating unit 2 is pivoted alternately from the first position P1 to the second position P2 and from the second position P2 to the first position P1.

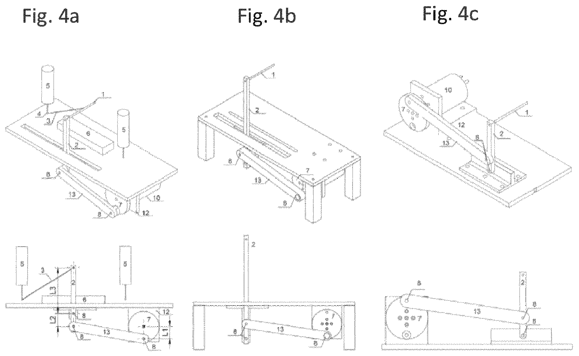

Fig. 4a shows a schematic drawing of a first variant for use in a device according to Figures 1a or 1b comprising a connecting strut 13 between the oscillating unit 2 and rotating unit 7. The upper section of Figure 4a shows a perspective view and the lower section a side view of the device. The device comprises a motor holder 12 and a motor unit. The motor holder 12 is a plate. The motor unit comprises a motor 10 fixed to a lower side of the stationary motor holder 12. The motor unit further comprises a rotating unit 7 that is arranged adjacent to the motor 10 at the lower side of the motor holder 12. The motor 10 is drivingly connected to the rotating unit 7 to rotate the rotating unit 7 around an axis of rotation. A connecting strut 13 is provided. The connecting strut 13 has an elongated shape with a first and a second end. The first end is pivotably connected to the rotating unit 7 via a first joint 8. The joint 8 is a swivel joint allowing rotational movement between the connecting strut 13 and the rotating unit 7. The joint 8 is distanced from the axis of rotation of the rotating unit 7. The second end of the connecting strut 13 is connected via a second swivel joint 8 to an oscillating unit 2. The oscillating unit 2 has an elongated shape and protrudes through a linear slit formed in the motor holder 12 such that a lower end of the oscillating unit is located at a lower side of the motor holder 12 and an upper end of the oscillating unit 2 is located at an upper side of the motor holder 12. The oscillating unit 2 is rotatably connected to a stationary part, for example, to the motor holder 12, about an oscillating axis via a pivot joint. The oscillating unit 2 has an elongated shape with a first area and a second area separated by the oscillating axis about which the oscillation unit 2 is at least partially pivotable. A fiber-picking unit 1 is located in the first area at a first end of the oscillating unit 2. Joint 8 connecting the connecting strut 13 to the oscillation unit 2 is located in the second area of the oscillating unit. Measured along a longitudinal axis of the oscillating unit 2, the distance from the oscillation axis to the fiber-picking unit 1 is less than the distance from joint 8, which connects the connecting strut 13 to the oscillation unit 2, to the fiber picking unit 1. In the shown example, the oscillating unit 2 is movably arranged in the linear slit. However, in other embodiments, the slit may be omitted. At the upper end of the oscillating unit 2, the fiber-picking unit 1 is provided. The fiber-picking unit 1 may be fixed by glue or welding, for example. The fiber-picking unit 1 is a rod extending essentially perpendicular to a longitudinal axis of the oscillating unit 2. On a top side of the motor holder 12 a receiving unit 6 with a receiving surface is arranged. The receiving surface is an upper surface of the receiving unit 6. Due to rotation of the rotating unit 7, the oscillating unit 2 is pivoted between a first position P1 and a second position P2. Two dispenser unit 5 for dispensing fluid are arranged on a top side of the motor holder. The pivoting movement of the oscillating unit 2 is guided by the connecting strut 13. When the fiber-picking unit 1 fixed to the oscillating unit 2 is in the first position P1, a droplet of fluid 4 dispensed by one of the dispensers 5 contacts the fiber-picking unit 1. When the fiber-picking unit 1 is pivoted from the first position P1 to the second position P2 a fiber 3 is formed and deposited on the receiving surface. When the fiber-picking unit 1 is in the second position P2, a droplet of fluid 4 dispensed by the other one of the dispensers 5 contacts the fiber-picking unit 1. When the fiber-picking unit 1 is pivoted from the second position P2 to the first position P1 another fiber 3 is formed and deposited on the receiving surface.

Fig. 4b shows a schematic drawing of a second variant for use in a device according to Figures 1a or 1b comprising a connecting strut 13 between the oscillating unit 2 and rotating unit 7. The device of Figure 4b is essentially identical to the device of Fig. 4a. The oscillating unit 2 is rotatably connected to a stationary part, for example, to the motor holder 12, about an oscillating axis via a pivot joint. The oscillating unit 2 has an elongated shape with a first area and a second area separated by the oscillating axis about which the oscillation unit 2 is at least partially pivotable. The fiber-picking unit 1 is located in the first area, in particular at a first end. Joint 8 connecting the connecting strut 13 to the oscillation unit 2 is also located in the first area. Measured along a longitudinal axis of the oscillating unit 2, the distance from joint 8, which connects the connecting strut 13 to the oscillation unit 2, to the fiber-picking unit 1 is less than the distance from the oscillation axis to the fiber-picking unit 1. The upper section of Figure 4b shows a perspective view and the lower section a side view of the device.

Fig. 4c shows a schematic drawing of a third variant for use in a device according to Figures 1a or 1b comprising a connecting strut 13 between the oscillating unit 2 and rotating unit 7. The upper section of Figure 4a shows a perspective view and the lower section a side view of the device. The device comprises a motor holder 12 and a motor unit. The motor unit comprises a motor 10 fixed to an upper side of the stationary motor holder 12. The motor unit further comprises a rotating unit 7 that is arranged adjacent to the motor 10 at the upper side of the motor holder 12. The motor 10 is drivingly connected to the rotating unit 7 to rotate the rotating unit 7 around an axis of rotation. A connecting strut 13 is provided. The connecting strut 13 has an elongated shape with a first and a second end. The first end is pivotably connected to the rotating unit 7 via a first joint 8. The joint 8 is a swivel joint allowing rotational movement. The joint 8 is distanced from the axis of rotation of the rotating unit 7. The second end of the connecting strut 13 is connected via a second swivel joint 8 to the oscillating unit 2. The oscillating unit 2 has an elongated shape. The oscillating unit 2 rotatably fixed to an upper side of the motor holder 12. The oscillating unit 2 is rotatably connected to a stationary part, for example, to the motor holder 12, about an oscillating axis via a pivot joint. The oscillating unit 2 has an elongated shape with a first and a second area separated by the oscillating axis about which the oscillation unit 2 is at least partially pivotable. A fiber-picking unit 1 is located in the first area of the oscillating unit 2. Joint 8 connecting the connecting strut 13 to the oscillation unit 2 is also located in the first area. Measured along a longitudinal axis of the oscillating unit 2, the distance from joint 8, which connects the connecting strut 13 to the oscillation unit 2, to the fiber picking unit 1 is less than the distance from the oscillation axis to the fiber picking unit 1. The fiber-picking unit 1 may be provided at the upper end of the oscillating unit 2. The fiber-picking unit 1 may be fixed by glue or welding, for example. The fiber-picking unit 1 is a rod extending essentially perpendicular to a longitudinal axis of the oscillating unit 2. On a top side of the motor holder 12 a receiving unit 6 with a receiving surface is arranged (not shown). The receiving surface is an upper surface of the receiving unit 6. Due to rotation of the rotating unit 7, the oscillating unit 2 is pivoted between a first position P1 and a second position P2. Two dispenser units 5 (not shown) for dispensing fluid are arranged on a top side of the motor holder 12. The pivoting movement of the oscillating unit 2 is guided by the connecting strut 13. When the fiber-picking unit 1 fixed to the oscillating unit 2 is in the first position P1, a droplet of fluid 4 dispensed by one of the dispensers 5 contacts the fiber-picking unit 1. When the fiber-picking unit 1 is pivoted from the first position P1 to the second position P2 a fiber 3 is formed and deposited on the receiving surface. When the fiber-picking unit 1 is in the second position P2, a droplet of fluid 4 dispensed by the other one of the dispensers 5 contacts the fiber-picking unit 1. When the fiber-picking unit 1 is pivoted from the second position P2 to the first position P1 another fiber 3 is formed and deposited on the receiving surface. An exemplary arrangement of the receiving unit 6, the fabricated fiber 3 and the dispensers 5 can be taken from Figure 4a.

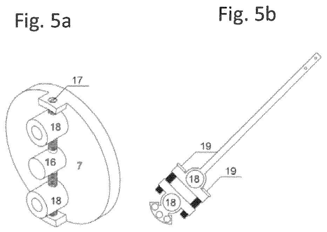

Figure 5a illustrates schematically an exemplary rotating unit 7 for use in the devices described above. Figure 5b illustrates schematically an exemplary oscillating unit 2 use in the devices described above.

A deflection angle of the oscillation unit 2 can be adjusted by varying the L2 and L1 lengths (as shown, for example, in Fig. 4a). L1 can be varied by using a rotating unit 7 according to Fig. 5a and oscillating unit according to Fig. 5b with adjustable joints 18. Via joints 18, at least one connecting unit 13 is fixed to the rotating unit 7/oscillating unit 2. The distance between the center of the rotating unit 7 and adjustable joint 18 can be varied by turning a screw 17. The screw 17 has left and right threads. The screw 17 is fixed inside a central element 16 such that on rotating it can rotate but cannot slide. The second adjustable joint 18 may be used for balance. The distance between the adjustable joints 18 in Figure 5b can be varied by turning screws 19. An oscillation radius can be controlled by connecting, for example gluing, the fiber-picking unit 1 at different distances from center of oscillation/rotation of the oscillating unit 2.

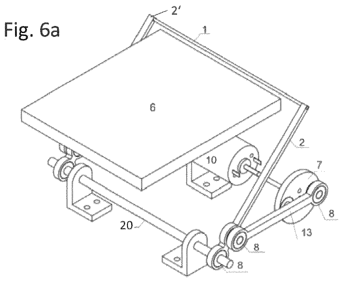

Figure 6a shows an exemplary embodiment of a device for fabrication of fibers comprising two oscillating units 2 and 2'. The mode of operation is essentially the same as that of the devices already described with regard to the previous figures. Thus, reference is made to the previous figures and in the following, in particular the differences are described below. The fiber-picking unit 1 can be attached to a single oscillating unit 2 by one end or by both ends to two oscillating unit as shown in Figure 6a to provide support for the fiber-picking unit 1, which can be deformed due to gravity or movement, and allows fabrication of larger samples and use of thinner and lighter fiber-picking units. A motor unit comprises a motor 10 fixed to stationary motor holder (not shown). The motor unit further comprises a rotating unit 7 that is driven by the motor 10 via a shaft. Thus, the motor 10 is drivingly connected to the rotating unit 7 to rotate the rotating unit 7 around an axis of rotation. A connecting strut 13 is provided. The connecting strut 13 has an elongated shape with a first and a second end. The first end is pivotably connected to the rotating unit 7 via a first joint 8. The joint 8 is a swivel joint allowing rotational movement. The joint 8 is distanced from the axis of rotation of the rotating unit 7. The second end of the connecting strut 13 is connected via a second swivel joint 8 to an oscillating unit 2. The oscillating unit 2 has an elongated shape. The oscillating unit 2 is rotatably fixed to the motor holder via a shaft 20. The connecting strut 13 is pivotably connected to the oscillating unit 2 distanced to the pivoting axis of the oscillating unit 2. At the upper end of the oscillating unit 2, a fiber-picking unit 1 is provided. The fiber-picking unit 1 may be fixed by glue or welding or by knot, for example. The fiber-picking unit 1 is a rod extending essentially perpendicular to a longitudinal axis of the oscillating unit 2. A second oscillating unit 2' is provided. An upper end of the second oscillating unit is connected to a second end of the fiber-picking unit 1. The second oscillating unit 2' is connected to a second end of the shaft 20. A second connecting strut (not shown) is connected to the oscillating unit 2'. The second connecting strut (not shown) is further connected to the motor shaft via a second rotating unit (not shown). The structure of the second rotating unit, the second connecting strut and the second oscillating unit corresponds to the structure of the first ones of these components. A receiving unit 6 with a receiving surface is provided. The receiving surface is an upper surface of the receiving unit 6. Due to rotation of the first rotating unit 7 and the second rotating unit, the oscillating units 2 and 2' are pivoted between a first position P1 and a second position P2. At least one dispenser unit 5 (not shown) for dispensing fluid is arranged such that the fiber-picking unit contacts dispensed fluid while in the first position P1. The pivoting movement of the oscillating units 2, 2' is guided by the connecting struts. When the fiber-picking unit 1 fixed to the oscillating units 2, 2' is in the first position P1, a droplet of fluid 4 dispensed by the dispensers 5 contacts the fiber-picking unit 1. When the fiber-picking unit 1 is pivoted from the first position P1 to the second position P2 a fiber 3 (not shown) is formed and deposited on the receiving surface.

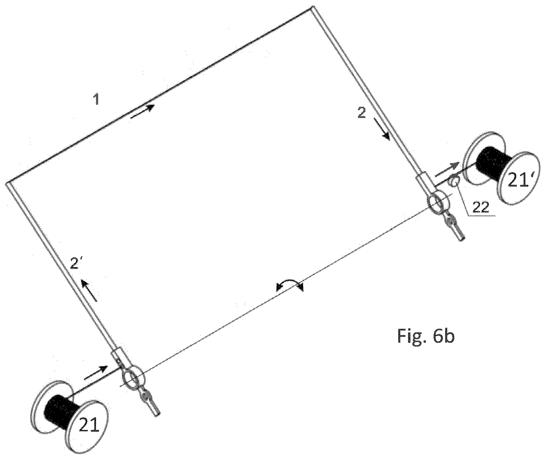

Figure 6b illustrates an exemplary assembly with two oscillating units 2 and 2' and a fiber-picking unit 1, wherein the fiber-picking unit 1 is a recoverable string. The oscillating units 2 and 2' each comprise a tube for guiding the string along their lengths. The string is wrapped around a first drum 21. The first drum may be arranged adjacent to a proximal end of the second oscillating unit 2'. The string is arranged in the tube of the second oscillating unit 2' and is guided by the tube to the distal end of the second oscillating unit 2'. The string is tensioned between the distal ends of oscillating units 2 and 2'. The string is arranged in the tube of the first oscillating unit 2 and wrapped around a second drum 21'. The second drum 21' may be arranged adjacent to a proximal end of the first oscillating unit 2. The tension of the string and its fiber-picking part may be provided by a tension wheel 22. The first and second drums may be rotatable to renew the string in the area of the fiber-picking unit 1. Thus, the rotation of first and second drum 21, 21' allows removal of a part of the fiber-picking unit 1, which may be contaminated with fluid, for example polymer.

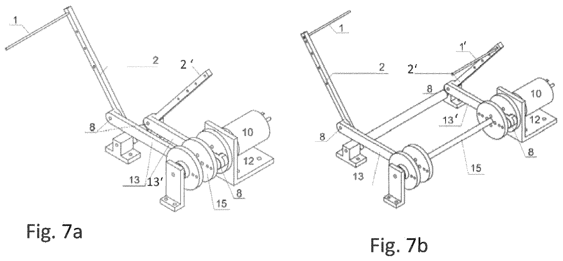

Figure 7a and Figure 7b each show a device for producing fibers, which corresponds essentially to the apparatus described with respect to the above figures. For reducing vibrations, the devices of Figures 7a and 7b comprise a crankshaft 15. Two oscillating units 2 and 2' are each connected to the crankshaft 15 via a connecting strut 13, 13', respectively. The connecting struts 13, 13' are each rotatably connected to the crankshaft 15 via rotating joints 8. The oscillating units 2 and 2' are each connected to the respective connecting strut 13, 13' via a rotating joint 8. The crankshaft 15 is rotated by a motor 10. The oscillating units 2 and 2' preferably do not have exactly the same shape. The second oscillating unit 2' can be hidden. Preferably, the moments of inertia of the first oscillating unit 2 and of the second oscillating unit 2' are similar to each other.

In Figure 7b, a second fiber-picking unit 1' is provided. The second fiber-picking unit 1' may be glued to the second oscillating unit 2'. The two oscillating units 2 and 2' move in antiphase to reduce vibrations.

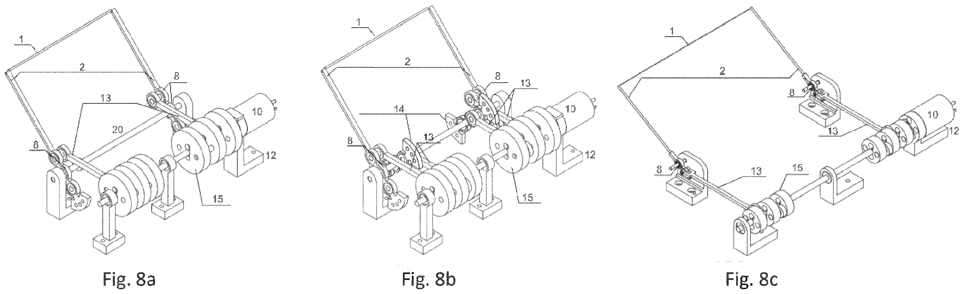

The setup, where the fiber-picking units 1 is connected by its two ends to two different oscillating units 2 and 2' (for example, as shown in Figs. 1a, 6a, 6b) can be combined with a setup with reduced vibrations (for example, as shown in Fig. 7a, 7b), in particular to allow deposition of fibers on large receiving unit 6. Such a device is shown in Figures 8a, 8b and 8c. The oscillating unit 2 is rotatably connected to a stationary part, for example, to the motor holder 12, about an oscillating axis via a pivot joint. In Figure 8a and 8b, the oscillating unit 2 has an elongated shape with a first area and a second area separated by the oscillating axis about which the oscillation unit 2 is at least partially pivotable. The fiber-picking unit 1 is located in the first area, in particular, at a first end. Joint 8 connecting the connecting strut 13 to the oscillation unit 2 is also located in the first area. Measured along a longitudinal axis of the oscillating unit 2, the distance from joint 8, which connects the connecting strut 13 to the oscillation unit 2, to the fiber picking unit 1 is less than the distance from the oscillation axis to the fiber picking unit 1. In Figure 8c, the oscillating unit 2 has an elongated shape with a first area and a second area separated by the oscillating axis about which the oscillation unit 2 is at least partially pivotable. The fiber-picking unit 1 is located in the first area, in particular at a first end. Joint 8 connecting the connecting strut 13 to the oscillation unit 2 is located in the second area. Measured along a longitudinal axis of the oscillating unit 2, the distance from the oscillation axis to the fiber picking unit 1 is less than the distance from joint 8, which connects the connecting strut 13 to the oscillation unit 2, to the fiber picking unit 1. In Figure 8a and 8c two parallel oscillating units 2 and 2' are provided. The device of Figure 8b may have four oscillating units 2, 2' 14, 14', wherein the shape of the oscillating units 2, 2'and 14, 14' may differ from each other. In particular, the device comprises two oscillating units 2 and 2', which are substantially identical, and two further oscillating units 14 and 14', which are arranged in parallel and are substantially identical but different from the oscillating units 2 and 2'. The oscillating units 2 and 2' have an elongated shape that is longer than the shape of oscillating units 14 and 14'. The oscillating units 14 and 14' are smaller than oscillating units 2 and 2'. The fiber-picking unit 1 is connected to oscillating units 2 and 2'. Two other oscillating units 14, 14' may be short. The moment of inertia of oscillating units 2 and 2'and oscillating units 14, 14' may be the same to reduce vibrations. Vibrations can also be reduced by balancing long and short oscillating units. With regard to recurring features already described in the previous figures, reference is made to these figures.

In Figure 8a and Figure 8c the oscillating units 2 and 2' moving in phase. In Figure 8b the oscillating units 2 and 2' moving in phase. In Figure 8b the oscillating units 2 and 2' moving in anti-phase with regard to oscillating units 14, 14'.

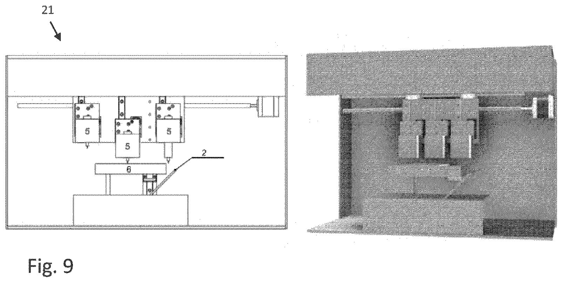

Figure 9 shows a 3D printer, in particular a 3D bioprinter, comprising a device according to one of the previous figures. On the left, a side view is schematically shown, on the right, a perspective view of the 3D printer 21 is shown. The 3D printer 21 comprises three movable dispenser units 5 to dispense a fluid 4. A receiving unit 6 is arranged below the dispensers 5. At least one oscillating unit 2 is arranged alternately pivotable below the dispensers 5 for generating a fiber and depositing the fiber on the receiving unit 6. Features already described in the previous figures may be applied to the device arranged in the shown 3d printer. Reference is made to the previous figures.

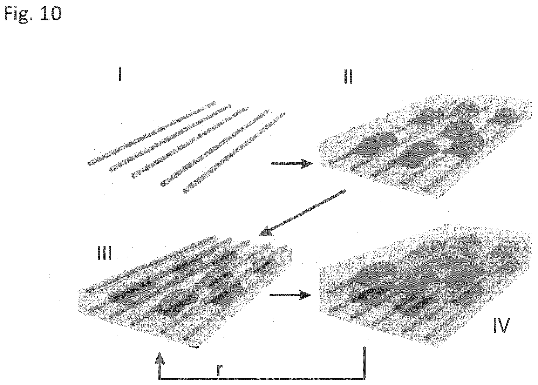

The oscillating unit 2 will move above the receiving unit 6, preferably comprising a rotating table, and deposit fibers on the top of a sample located on the rotating table. Use of rotating table may allow deposition of fibers in different directions. One or more force sensor(s) may be integrated in the rotating table to measure mass of deposited fibers and control the fiber spinning process. The obtained aligned nano-micro-macro-sized fibers can be used as scaffold for cell culturing. This is particularly advantageous for biofabrication of skeletal and cardiac muscle tissues as well as for other tissues with fibrous structure such as skin or uniaxial aligned cells. For this, cells can be adsorbed or printed on the surface of layer formed by fibers. This is shown, for example, in Figure 10. In a first step I fibers are deposited. Next, in a step II, cells are extrusion printed, for example, in a 3d bioprinter. In a subsequent step III, another layer of fibers is deposited. In step III, the fibers may be deposited in another orientation. In a following step IV, cells are extrusion printed again and deposited on the previous layer of fibers. Steps III and IV may be repeated (as illustrated by the arrow r), in particular n times, until a target product is generated. Several alternating layers of fibers and cells can be deposited. The fabricated structure can be matured in a bioreactor.

In the following, three examples of fabrication of fibers are described. The features described below may be applied to the embodiments shown in the previous figures.

### Example 1. Fabrication of hydrophobic fibers by spinning from solution.

The fluid 4 is a 10 - 20 % w/v solution of polycaprolactone (Sigma Aldrich, Mn = 85 kDa) in chloroform and is prepared by dissolution of solid polymer using a magnetic stirrer. The solutions were transferred in a 5 ml syringe as dispenser 5. A 27G needle is used. For example, a device as shown in Fig. 4b may be used for production of fibers. Alternatively, the setup of Fig. 1a, 1b, 2 4a, 4c, 3a, 3b, 3c, 3d, 5a, 5b, 6a, 6b, 7a, 7b, 8a, 9 and/or 8b, 8c could be used. The motor runs at a speed of 300 rpm to drive oscillating unit 2 with fiber-picking unit 1. The polymer solution may be dispensed from the syringe by manually or automatically applying pressure close to fiber-picking unit 1. The freestanding fibers may be deposited between two metallic rod-like holders or any other object such as, for example, a finger.

### Example 2: Fabrication of fibers by spinning from melt.

The fluid 4 is melt of TPU Flex Semisoft - Shore hardness A 88. The fluid 4 is extruded in a Microswiss extruder controlled by Arduino-based controlled with Marlin firmware, which is usually used for controlling 3D printers. For example, the setup illustrated in Fig. 4b may be used for production of fibers. Alternatively, the setup of Fig. 1a, 1b, 2 4a, 4c, 3a, 3b, 3c, 3d, 5a, 5b, 6a, 6b, 7a, 7b, 9, 8a and/or 8b, 8c could be used. The motor 10 is driven with at a speed of 100 rpm to drive oscillating unit 2 with fiber-picking unit 1. The polymer melt is extruded at 220°C by positioning the extruder close to fiber-picking unit 1 and produced fibers 3 are deposited between two metallic rod-like holders and/or on a receiving surface.

### Examples 3. Fabrication of hydrophilic fibers by spinning from solution.

The fluid 4 is 3 - 10 % w/v solution of polyethylene glycol (Sigma Aldrich, Mn = 1000 kDa) in chloroform and is prepared by dissolution of solid polymer using a magnetic stirrer. The solutions are transferred in a 5 ml syringe. The 27G needle is used. The setup illustrated in Figure 7a is exemplarily used for production of fibers. Alternatively, the setup of Fig. Fig. 1a, 1b, 2 4a, 4b, 4c, 3a, 3b, 3c, 3d, 5a, 5b, 6a, 6b, 7b, 8a and/or 8b, 8c and/or 9 could be used. The motor runs at a speed of 300 rpm to drive the oscillating unit 2 with fiber-picking unit 1. The polymer solution is dispensed from the syringe by manually or automatically applying a pressure close to fiber-picking unit 1.

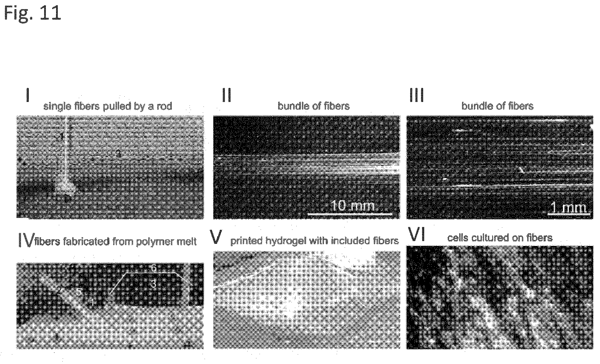

Figure 11 shows six examples of produced fibers. Examples of fibers produced using 15% w/v solution of polycaprolactone (Mn = 85 kDa) in chloroform is shown in I, II, III. IV shows an example of fibers fabricated using polymer melt (TPU Flex Semisoft - Shore hardness A 88 filament). V shows hydrogel with embedded fibers produced using a combination of fiber spinning and 3D printing. VI shows mouse fibroblast cells on the surface of fibers.
1, - first fiber-picking unit
1' - second fiber-picking unit
2 - first oscillating unit
2' - second oscillating unit
3 - fiber
4-fluid
5 - first dispenser unit
6 - receiving unit
61 - receiving surface
7 - rotating unit
8 - flexible joint
9 - guiding slot
10 - motor
11 - pin
111 - needles
12 - motor holder
13 - connecting unit
14, 14' - small oscillating unit
15 - crankshaft
16 - central element for fixation of screw
17 - screw with left and right threads fixed, which is able to rotate around central element fixed on the rotating unit
18 - joint, which is moved by adjustment of a screw, for connecting of the connecting rod
19 - adjustment screw
20 - shaft
21 - 3D printer

## Claims

1. Device for fabrication of fibers (3), the device comprising
- a receiving unit (6) having a receiving surface for receiving fabricated fibers (3)
- a first dispenser unit (5) for dispensing a fluid,
- a motor unit comprising a rotating unit (7) and a motor (10), wherein the motor (10) is configured to drive the rotating unit (7),
**characterized by**
- a first oscillating unit (2) arranged alternately pivotable with respect to the receiving surface and connected to the rotating unit (7) such that the rotating unit (7) drives the first oscillating unit (2) to oscillate,
the first oscillating unit (2) comprising a fiber picking unit (1), the first oscillating unit (2) configured to move the fiber picking unit (1) alternately relative to the receiving surface between a first (P1) and a second position (P2), wherein in the first position (PI), the fiber picking unit (1) is arranged adjacent to the first dispenser unit (5) such that fluid (4) dispensed by the first dispenser unit (5) contacts the fiber picking unit (1), wherein the fiber picking unit (1) is configured to form a fiber (3) when pivoting from the first position (P1) to the second position (P2).

2. Device according to claim 1, wherein the first and the second position are located on an imaginary circle having a center corresponding to the pivot axis of the first oscillating unit (2) and wherein the movement of the fiber picking unit (1) from the first to the second position (P2) is in a first rotational direction and the movement from the second (P2) to the first position (P1) is in a second rotational direction opposite to the first rotational movement.

3. Device according to one of the previous claims, further comprising a second dispenser unit (5) for dispensing a fluid, the second dispenser unit (5) being arranged adjacent to a travel path of the fiber picking unit (1) such that fluid dispensed by the second dispenser unit can contact the fiber picking unit (1) when the fiber picking unit (1) is in a third position.

4. Device according to one of the previous claims, wherein the fiber picking unit (1) is configured to deposit the formed fibers (3) on the receiving surface.

5. Device according to one of the previous claims, wherein the fiber picking unit (1) is a rod, in particular comprising at least one surface having a multitude protruding pins and/or needles (11) and/or cones.

6. Device according to one of the previous claims, wherein the first and/or the second dispenser unit (5) comprises at least one nozzle for dispensing the fluid (4), in particular comprising a multitude of nozzles.

7. Device according to one of the previous claims, wherein electric voltage may be applied between the fiber picking unit (1) and the first and/or second dispenser unit (5) to stimulate transfer of fluid (4) from the first and/or second dispenser unit (5) to the fiber picking unit (1).

8. Device according to one of the previous claims, wherein
the first oscillating unit (2) comprises an essentially linear guiding slot and a pin is slidably arranged in the guiding slot, wherein the pin is fixed to a stationary part or fixed to the rotating unit (7) of the motor unit
and/or
the first oscillating unit (2) is rotatably fixed to the rotating unit (7) and comprises a pin slideably arranged in a semicircular guiding slot.

9. Device according to one of the previous claims, further comprising a first connecting strut having a first end rotatably fixed to the first oscillating unit (2) and a second end rotatably fixed to the rotating unit (7).

10. Device according to one of the previous claims,
wherein the rotating unit (7) comprises at least one adjustable joint connecting the rotating unit (7) to the first oscillating unit (2) and/or the first connecting strut for varying a minimal distance between the adjustable joint and a rotating axis of the rotating unit (7);
and/or
wherein the first oscillating unit (2) comprises at least one adjustable joint connecting the rotating unit (7) to the first oscillating unit (2) for varying a minimal distance between the adjustable joint and the fiber picking unit (1).

11. Device according to one of the previous claims, further comprising a second oscillating unit (2');
in particular wherein the fiber picking unit (1) is a string, wherein the string is spanned between the first and second oscillating units (2, 2'), in particular wherein the string is a thread or a wire, in particular wherein the first and second oscillating units (2, 2') each comprise at least one tube for receiving and guiding the string.

12. Device according to one of the previous claims, further comprising a second connecting strut (13), wherein the rotating unit (7) is a shaft and the second connecting strut is rotatably connected to the shaft, and the fiber picking unit (1) has a first end connected to the first oscillating unit (2) and a second end connected to the second oscillating unit (2').

13. Device according to one of the previous claims, further comprising a second oscillating unit (2') and a second connecting strut, the second oscillating unit (2') and the second connecting strut being rotatably connected, wherein the rotating unit (7) is a crankshaft and the first and second connecting struts are each rotatably connected to the crankshaft to move in antiphase or in phase; the device in particular further comprising a third and/or a fourth oscillating unit each connected to the rotating element via a connecting strut, wherein the moments of inertia of the first oscillating unit (2) and of the third oscillating unit and/or of the second oscillating unit (2') and of the fourth oscillating unit are essentially the same to reduce vibrations.

14. 3D-printer comprising a device according to one of the previous claims.

15. Method for fabrication of fibers (3), the method comprising the following steps:
- Providing a device according to one of the claims 1 to 16,
- Pivoting the fiber picking unit (1) to the first position in a first direction adjacent to the first dispenser unit (5) so that the fluid (4) dispensed from the first dispenser unit (5) contacts the fiber picking unit (1),
- Pivoting the fiber picking unit (1) to the second position (P2) in a second direction opposite to the first direction, thereby forming a fiber and depositing the formed fiber on the receiving surface.
